# DEMANDE DE BREVET EUROPEEN

(11) **EP 1 815 824 A1**
(43) Date de publication de la demande: **08.08.2007**
(21) Numéro de dépôt: 07356011.2
(22) Date de dépôt: 31.01.2007
(51) Int. Cl.: A61F 2/38

(54) **Implant tibial a tige offset**

(30) Priorité: 01.02.2006 FR 0600913
(71) Demandeur: TORNIER, 38330 Saint-Ismier (FR)
(72) Inventeur: Rochetin, Olivier, 42130 Marcilly le Chatel (FR)
(74) Mandataire: Grand, Guillaume

(57) **Abrégé**

Cet implant tibial (1) comprend un plateau (10), une tige (30) et un élément adaptateur (40) interposé fixement entre le plateau et la tige. Pour permettre de décaler un second axe tibial (31) associé à la tige par rapport à un autre premier axe tibial (11) associé au plateau, l'élément adaptateur comporte des parties proximale (43) et distale (45) à même d'être respectivement assemblées au plateau et à la tige, de manière respectivement centrée sur les deux axes tibiaux. Pour faciliter la mise en place de cet implant dans un tibia, améliorer son comportement d'un point de vue anatomique, et offrir une grande liberté de réglage lors de son assemblage, la partie proximale de l'élément adaptateur est munie de moyens d'indexage angulaire (47), autour du premier axe tibial, par rapport au plateau, adaptés pour régler de manière discrète le positionnement à la fois médio-latéral et antéro-postérieur du second axe tibial par rapport au premier axe tibial lorsqu'une partie (17) du plateau, qui s'étend directement en saillie axiale depuis la surface distale (12B) d'une plaque principale (12) du plateau, est logée en totalité dans un orifice proximal (42) délimité par la partie proximale de l'élément adaptateur.

## Description

La présente invention concerne un implant tibial, du type comportant un plateau à fixer à l'extrémité supérieure du tibia et une tige à introduire dans le canal médullaire du tibia, ce plateau pouvant être décentré ou décalé par rapport à l'axe longitudinal de la tige. Ce type d'implant est souvent qualifié d'implant tibial à « tige offset ».

Un implant de ce type appartient généralement à une prothèse de genou et est alors associé, d'une part, à un implant fémoral fixé à l'extrémité inférieure du fémur et, d'autre part, à un patin interposé entre l'implant fémoral, contre lequel le patin est articulé, et le plateau, contre lequel le patin est prévu soit fixe, soit mobile.

L'utilisation d'un tel implant tibial à tige offest vise à optimiser le positionnement médio-latéral et antéro-postérieur du plateau, plaqué contre l'extrémité réséquée du tibia, par rapport au canal médullaire tibial, étant remarqué que ce canal impose la position de la tige de l'implant. En effet, en fonction du genou opéré, il est souhaitable que, tout en étant parallèles, l'axe longitudinal de la tige et l'axe central du plateau soient décalés l'un par rapport à l'autre, en pratique de quelques millimètres au maximum.

Actuellement, les implants tibiaux de ce type comportent une tige offset rapportée à l'extrémité distale d'une quille du plateau, c'est-à-dire à l'extrémité distale d'un prolongement de matière allongé qui s'étend, de manière centrée sur l'axe central du plateau, depuis la face distale du plateau et qui est destinée à être introduit dans l'extrémité épiphysaire du canal médullaire du tibia. Cette quille s'étend généralement en longueur sur 20 à 40 mm, en fonction des implants. Pour décaler l'axe de la tige par rapport à celui de la quille, soit la partie proximale de la tige présente une forme coudée de manière que son extrémité proximale, rapportée sur la quille tibiale, est décalée par rapport à sa partie distale reçue dans la partie diaphysaire du canal médullaire, comme proposé dans US-A-6,146,424 ou US-A-5,290,313, soit un élément adaptateur de forme coudée est interposé fixement entre l'extrémité distale de la quille et l'extrémité proximale de la tige, comme proposé dans US-A-6,162,255 ou EP-A-0 853 930. Cette seconde solution présente l'avantage, par rapport à la première, de proposer un implant tibial modulaire, dans lequel un même couple plateau/tige peut être relié par un élément adaptateur choisi parmi une gamme d'éléments adaptateurs présentant des valeurs de décalage respectives différentes. Cependant, ces deux solutions ont pour inconvénient de situer la zone effective du décalage des deux axes à une profondeur significative du canal médullaire tibial. Le chirurgien est alors obligé de préparer au préalable ce canal, en enlevant de la matière osseuse dans une zone du tibia assez fragile. De plus, en service, la répartition des efforts développés au niveau de cette zone de décalage au sein de la partie épiphysaire du canal médullaire s'écarte du comportement anatomique constaté avec un tibia sain.

US-B-6,214,052 propose un implant tibial à tige offset réversible. Cet implant inclut un élément adaptateur qui relie un plateau tibial et la tige, en étant assemblé au plateau dans deux configurations possibles, selon qu'on souhaite décaler l'axe tibial de la tige à droite ou à gauche par rapport à l'axe tibial associé au plateau. L'élément adaptateur délimite à cet effet un orifice proximal dans lequel est logé un renflement saillant de la face distale du plateau, sans interposition d'une quille telle que celle évoquée ci-dessus. Toutefois, en pratique, la réversibilité gauche/droite de l'assemblage entre le plateau et l'élément adaptateur est obtenue en prévoyant que les sections transversales respectives du renflement et de l'orifice sont oblongues, ce qui n'autorise aucune liberté de réglage pour le positionnement relatif des deux axes tibiaux, sauf la liberté de choix entre les deux configurations opposées gauche/droite. Un très grand nombre d'éléments adaptateurs, avec des configurations respectives différentes du décalage angulaire entre leurs parties proximale et distale, doit alors être prévu à la disposition du chirurgien, ce qui induit des problèmes de coûts, de logistique et d'efficacité.

Le but de la présente invention est de proposer un implant tibial modulaire à tige offset, qui soit plus facile à implanter dans le tibia et qui se comporte de manière plus satisfaisante d'un point de vue anatomique, tout en offrant une grande liberté de réglage lors de son assemblage.
A cet effet, l'invention a pour objet un implant tibial tel que défini à la revendication 1.

Avec l'implant tibial selon l'invention, l'élément adaptateur, qui permet le décalage effectif entre les deux axes tibiaux de l'implant, est assemblé au plus près du plateau, c'est-à-dire à proximité immédiate de la surface distale de la plaque de ce plateau. Autrement dit, contrairement aux implants tibiaux relevant de l'art antérieur, aucune quille n'est interposée entre cette plaque et l'élément adaptateur. En outre, grâce aux moyens d'indexage angulaire de l'élément adaptateur, le chirurgien peut choisir, pour une valeur donnée du décalage entre les premier et second axes tibiaux, propre à l'élément adaptateur utilisé, le positionnement du second axe tibial tout autour du premier axe tibial. Ainsi, sans changer d'élément adaptateur, le chirurgien ajuste librement, de manière discrète, le positionnement relatif entre les premier et second axes tibiaux dans tout le plan horizontal, c'est-à-dire suivant des directions à la fois médio-latérale et antéro-postérieure.

Lorsque les composants de l'implant selon l'invention sont assemblés les uns aux autres et implantés au niveau d'un tibia, la surface distale de la plaque vient s'appuyer fermement contre l'extrémité supérieure du tibia, préalablement préparée, tandis que l'élément adaptateur est situé dans le débouché de l'extrémité épiphysaire du canal médullaire tibial, le reste de ce canal recevant la tige. Comme l'élément adaptateur est assemblé le plus près possible de la plaque du plateau, la préparation osseuse du canal médullaire concerne essentiellement, voire exclusivement, l'extrémité épiphysaire de ce canal, c'est-à-dire une zone facilement accessible au chirurgien et présentant une certaine robustesse. De plus, la répartition des efforts au niveau de l'extrémité épiphysaire du tibia muni de l'implant, lorsque cet os est sollicité, est alors sensiblement conforme aux constatations anatomiques sur un tibia sain.

Des caractéristiques avantageuses de l'implant selon l'invention sont énoncées aux revendications dépendantes 2 à 4.

Pour faciliter l'assemblage du plateau et de l'élément adaptateur, ainsi que pour offrir au chirurgien une matérialisation du premier axe tibial associé au plateau, la partie précitée du plateau, à même d'être logée dans l'orifice proximal, présente une forme essentiellement annulaire, centrée sur le premier axe tibial.

En pratique, pour limiter l'encombrement axial de la partie proximale de l'élément adaptateur, tout en garantissant un assemblage efficace entre cette partie et le plateau, la dimension axiale de la partie du plateau est inférieure à 15 mm, de préférence égale à 10 mm.

Une forme de réalisation particulièrement compacte de l'élément adaptateur est définie à la revendication dépendante 7. On notera que cette disposition peut être envisagée de manière indépendante à la nature précise de l'assemblage entre le plateau et la partie proximale de l'élément adaptateur. En particulier, cette disposition présente un réel intérêt de compacité et de fiabilité même si l'élément adaptateur n'est pas assemblé au plus près de la surface distale de la plaque du plateau.

En combinaison avec cette forme de réalisation compacte, l'implant tibial selon l'invention comprend en outre un élément de verrouillage tel que défini à la revendication 8. De la sorte, par la seule manipulation de cet élément de verrouillage, le chirurgien immobilise les uns par rapport aux autres les composants de l'implant tibial assemblés les uns aux autres, cet élément de verrouillage occupant alors avantageusement un espace laissé libre dans l'orifice proximal de l'élément adaptateur.

Les dispositions énoncées à la revendication 9 facilitent les gestes du chirurgien, en lui permettant de manipuler l'élément de verrouillage depuis le côté proximal du plateau.

Une autre caractéristique avantageuse de l'implant est énoncée à la revendication 10.

L'invention sera mieux comprise à la lecture de la description qui va suivre, donnée uniquement à titre d'exemple et faite en se référant aux dessins sur lesquels:
- les figures 1 et 2 sont des vues éclatées en perspective, sous des angles de vues respectifs différents, d'un implant tibial selon l'invention ; et
- la figure 3 est une coupe longitudinale partielle de certains des composants de l'implant des figures 1 et 2, assemblés les uns aux autres.

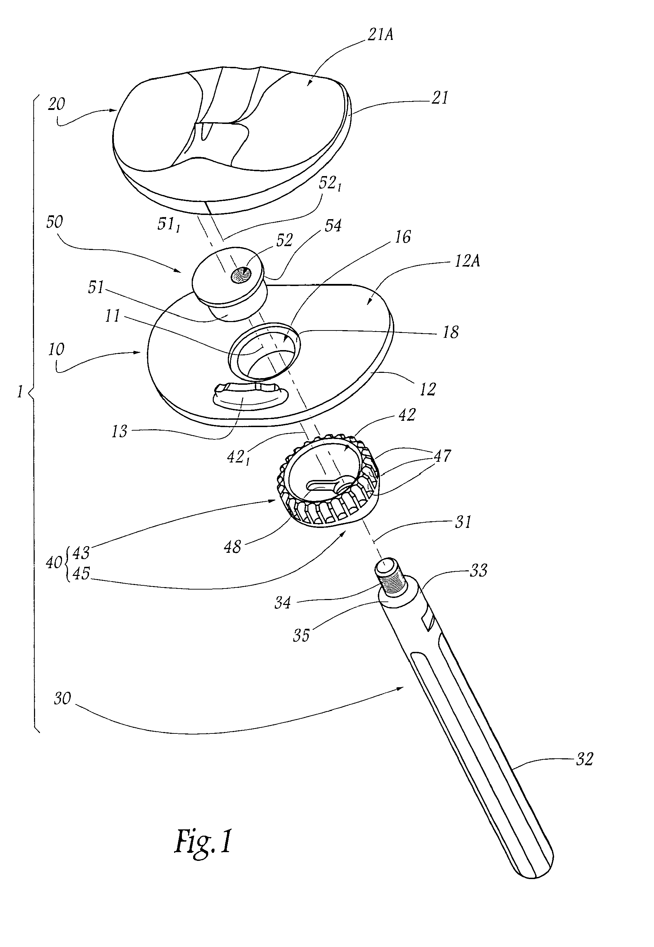

Sur les figures 1 à 3 est représenté un implant tibial 1 appartenant à une prothèse de genou d'un être humain. Par commodité, la suite de la description est orientée par rapport au genou d'un patient se tenant debout de sorte que le terme « proximal » désigne une direction dirigée vers l'articulation du genou tandis que le terme « distal » désigne une direction de sens opposé. De même, les termes « supérieur » et « haut » et, à l'opposé, « inférieur » et « bas » s'entendent par rapport à une direction globalement verticale par rapport au sol sur lequel le patient se tient debout, avec son tibia s'étendant ainsi de manière sensiblement verticale.

L'implant 1 comporte cinq composants distincts, à même d'être assemblés les uns aux autres de manière que l'implant présente une configuration assemblée permettant son implantation d'un seul tenant au niveau du tibia, non représenté, à savoir un plateau 10, un patin 20, une tige 30, un élément adaptateur 40 et un élément de verrouillage 50. Ces composants vont être successivement décrits ci-dessous.

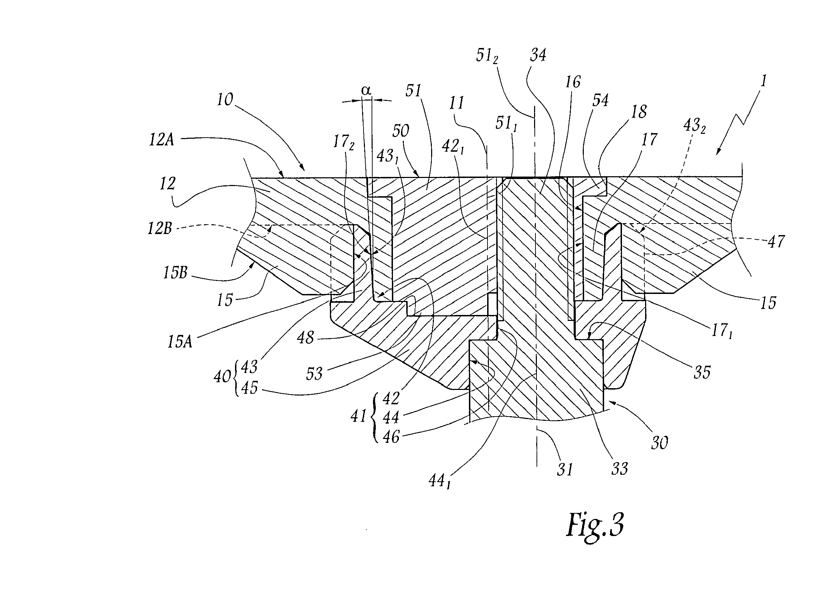

Le plateau 10 définit un axe central 11 essentiellement vertical et comporte une plaque principale 12 globalement plane et s'étendant de manière sensiblement perpendiculaire à l'axe 11, en étant globalement centrée sur cet axe. Comme représenté sur les figures 1 et 3, la plaque 12 délimite ainsi une surface proximale plane 12A contre laquelle la surface distale 21B du corps 21 du patin 20 est prévue pour s'appuyer et glisser suivant un contact plan/plan. Cette liaison mobile entre le plateau et le patin est guidée par une patte proximale 13 s'étendant en saillie, suivant l'axe 11, depuis la surface 12A de la plaque 12, en étant de préférence venue de matière avec cette dernière. Cette patte présente, en coupe horizontale, un profil incurvé sensiblement centré sur l'axe 11. Lorsque le patin est plaqué contre le plateau, la patte 13 est reçue dans une rainure associée 22 creusée dans la surface distale 21B du corps 21, de sorte que les déplacements relatifs entre le patin et le plateau suivent une trajectoire incurvée liée au coulissement de la patte 13 le long de la rainure 22.

Le corps 21 du patin 20 délimite une surface proximale 21A définissant des zones articulaires vis-à-vis d'un composant fémoral, non représenté, de la prothèse de genou ou de l'extrémité inférieure anatomique du fémur du patient.

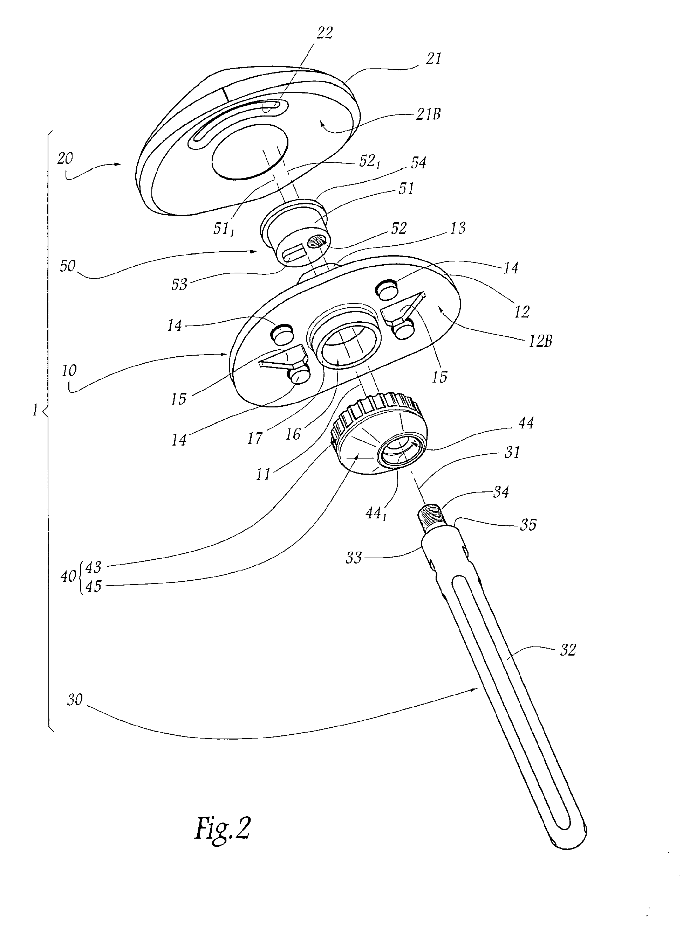

Comme représenté sur la figure 2, la plaque 12 délimite une surface distale plane 12B destinée à être plaquée fermement en appui contre l'extrémité supérieure du tibia, préalablement préparée, par exemple par résection, de manière à former un contact plan/plan. Pour améliorer l'ancrage osseux de la plaque contre cette extrémité tibiale, la plaque est munie de plots 14 s'étendant radialement en saillie, suivant l'axe 11, depuis la surface distale 12B. En plus des plots 14, la plaque 12 est munie de deux ailettes 15 qui s'étendent en saillie, suivant l'axe 11, depuis respectivement les parties médiale et latérale de la surface distale 12B. Ces ailettes 15 sont sensiblement symétriques par rapport à l'axe 11 et appartiennent globalement à un plan vertical médio-latéral correspondant au plan de coupe de la figure 3. L'un des intérêts principal de ces ailettes sera expliqué ci-après, étant remarqué que chaque ailette comporte, d'une part, sur son côté tourné vers l'axe 11, un bord 15A sensiblement parallèle à cet axe, et, d'autre part, sur son côté opposé, un bord incliné 15B qui s'éloigne de l'axe 11 en direction de la plaque 12. La forme inclinée, ou plus généralement évasée, des bords 15B est dimensionnée pour s'adapter au débouché épiphysaire du canal médullaire tibial, de manière à caler le plateau 10 au niveau de ce débouché par un effet de coin.

La plaque 12 délimite, dans sa partie centrale traversée par l'axe 11, un orifice traversant 16 qui, dans l'exemple considéré, est sensiblement centré sur l'axe 11. Cet orifice présente une forme globalement cylindrique, à base circulaire et d'axe 11, et s'étend sur toute l'épaisseur de la plaque, c'est-à-dire débouche à la fois sur, vers le haut, la surface proximale 12A et sur, vers le bas, la surface distale 12B.

Sur le côté distal du plateau 10, le débouché de l'orifice traversant 16 n'est pas au raz de la surface 12B de la plaque 12, mais est entouré, sur toute sa périphérie, d'une partie annulaire 17 qui s'étend en saillie vers le bas, suivant l'axe 11, depuis la surface 12B, en étant de préférence venue de matière avec la plaque 12. Ainsi, cette partie annulaire 17 est sensiblement centrée sur l'axe 11 et délimite une surface intérieure 17₁ sensiblement cylindrique, à base circulaire d'axe 11. La partie annulaire 17 délimite également une surface extérieure 17₂ sensiblement tronconique, d'axe 11 et convergente vers le bas, avec un demi-angle au sommet noté α sur la figure 3.

La tige 30 définit un second axe tibial 31 qui, dans l'exemple considéré, correspond à l'axe central longitudinal de cette tige. La tige 30 comporte une partie distale 32 extérieurement cannelée, destinée à être introduite dans le canal médullaire du tibia, essentiellement au niveau de sa partie diaphysaire. La tige 30 comporte également une partie proximale 33 étagée suivant l'axe 31. Plus précisément, cette partie 33 comprend un pion extrême proximal 34 extérieurement fileté et présentant un diamètre inférieur à celui du reste cylindrique de la partie 33, relié à la partie de tige distale 32. Un épaulement radial 35 relie ainsi le pion 34 au reste de la partie de tige 33.

L'élément adaptateur 40 est adapté pour relier, mécaniquement et de manière fixe, le plateau 10 et la tige 30, de manière que les axes tibiaux 11 et 31 soient sensiblement parallèles l'un à l'autre, tout en étant radialement décalés d'une valeur pré-déterminée, de l'ordre de quelques millimètres en pratique. A cet effet, l'élément 40 délimite intérieurement un orifice 41 qui traverse de part en part l'élément 40 suivant la direction des axes 11 et 31. Cet orifice 41 se compose en fait d'un orifice proximal 42, délimité par une partie proximale 43 de l'élément 40 et centré sur un axe 42₁, et d'un orifice distal 44, délimité par une partie distale 45 de l'élément 40 et centré sur un axe 44₁ parallèle à l'axe 42₁. Un passage 46, centré sur l'axe 44₁, relie le fond distal de l'orifice 42 au fond proximal de l'orifice 44. Dans l'exemple considéré aux figures, la partie proximale 43 présente une forme globalement tubulaire, centrée sur l'axe 42₁ et délimitant intérieurement l'orifice 42.

L'orifice 42 est dimensionné pour recevoir, de manière complémentaire, la partie annulaire saillante 17 du plateau 10, comme représenté sur la figure 3. A cet effet, la surface intérieure 43₁ de la partie tubulaire 43 présente une forme tronconique d'axe 42₁, sensiblement complémentaire de la surface extérieure 17₂ de la partie annulaire 17. L'assemblage du plateau 10 et de l'élément 40 consiste ainsi à introduire la partie 17 dans l'orifice 42, de manière centrée sur l'axe 11, étant remarqué, que, comme représenté sur la figure 3, l'axe 42₁ est alors sensiblement confondu avec l'axe 11.

L'orifice distal 44 est, quant à lui, dimensionné pour permettre l'assemblage de la tige 30. A cet effet, l'orifice 44 présente une forme cylindrique d'axe 44₁, sensiblement complémentaire de la partie de tige proximale 33, tandis que le passage 46 présente une section sensiblement complémentaire de celle du pion proximal extrême 34. De la sorte, l'assemblage de la tige 30 avec l'élément 40 consiste à introduire la partie de tige proximale 33 à l'intérieur de l'orifice 44, avec le pion extrême 34 reçu dans le passage 46, de manière centrée sur l'axe 31, étant remarqué que, comme représenté à la figure 3, l'axe 44₂ est alors sensiblement confondu avec l'axe 31.

La partie proximale tubulaire 43 présente une surface extérieure 43₂ crantée, c'est-à-dire munie d'une succession de crans 47 répartis de manière sensiblement uniforme suivant sa périphérie, chaque cran s'étendant en longueur suivant une direction parallèle à l'axe 42₁. Chaque cran 47 est dimensionné pour recevoir, de manière sensiblement complémentaire, le bord 15A des ailettes 15 lorsque le plateau 10 et l'élément 40 sont assemblés l'un à l'autre, étant remarqué que la distance radiale séparant deux crans diamétralement opposés correspond sensiblement à la distance radiale séparant les bords 15A des deux ailettes. En pratique, la section transversale de chaque cran présente, par exemple, une forme de U. Dans l'exemple considéré, vingt-quatre crans 47 sont répartis sur la surface extérieure 43₂ de sorte que deux crans successifs sont séparés d'un arc angulaire de 15°.

La coopération des crans 47 et des ailettes 15 permet d'indexer la position angulaire de l'élément 40 par rapport au plateau 10, autour de l'axe 11. En effet, lorsque le plateau 10 et l'élément 40 sont assemblés l'un à l'autre, un couple de deux crans 47 diamétralement opposés est mis en prise avec les bords 15A des ailettes 15, comme sur la figure 3, ce qui fixe la position angulaire de l'élément 40 autour de l'axe 11. Si l'on souhaite décaler angulairement cette position, on utilise l'un des couples de crans opposés qui suit le couple précité suivant la périphérie extérieure de la partie 43. Autrement dit, la position angulaire de l'élément 40 par rapport au plateau 10 est indexable de manière discrète avec un pas de 15° dans l'exemple considéré, étant remarqué qu'à chaque position indexée de l'élément 40, par rapport à l'axe 11, correspond une position correspondante de l'axe 44₁, autrement dit de l'axe 31 lorsque la tige 30 est assemblée à l'élément 40. De manière générale, on comprend que les crans 47 permettent ainsi de régler de manière discrète la position angulaire de l'axe 31 tout autour de l'axe 11. Le nombre de positions ainsi indexées dépend de la valeur du pas angulaire des crans 47, étant noté qu'une valeur de pas strictement inférieur à 180° implique que l'élément adaptateur 40 est indexable dans au moins trois positions angulaires autour de la partie annulaire 17, qui correspondent respectivement à au moins trois positionnements différents suivant des directions médio-latérale et antéro-postérieure.

L'élément de verrouillage 50 comporte un corps principal 51 ayant une forme globalement cylindrique, d'axe central 51₁ et sensiblement complémentaire de l'orifice 16 délimité dans le plateau 10. Ce corps 51 est muni d'un trou traversant taraudé 52 d'axe longitudinale 52₁ sensiblement parallèle à l'axe 51₁, en étant décalé par rapport à ce dernier. Le trou 52 est adapté pour recevoir, par vissage, le pion fileté 34 de la partie de tige proximale 33.

L'assemblage des composants de l'implant 1 est le suivant.

Dans un premier temps, on assemble le plateau 10 et la tige 30 en interposant entre eux l'élément 40. En pratique, le chirurgien dispose de plusieurs éléments 40, présentant respectivement des décalages radiaux respectifs, entre les axes 42₁ et 44₁ de leurs orifices proximal 42 et distal 44, de valeurs différentes, formant typiquement une série discrète de valeurs standardisées, tels que 1, 2, 3, 4 et 5 mm. Avantageusement, un élément adaptateur 40 à décalage nul peut être également prévu dans la gamme des éléments adaptateurs à disposition du chirurgien, ce qui revient alors disposer d'un élément adaptateur dont les axes 42₁ et 44₁ s'étendent dans le prolongement rectiligne l'un de l'autre.

Après l'avoir choisi, le chirurgien rapporte l'élément adaptateur 40 sur la surface distale 12B de la plaque 12 du plateau 10 : la partie annulaire 17 est axialement introduite dans l'orifice proximal 42, tandis que les bords 15A des ailettes 15 sont axialement introduits dans deux crans diamétralement opposés 47 choisis par le chirurgien selon le positionnement angulaire que ce dernier souhaite entre le plateau et la tige 30 à assembler juste après. Ce mouvement de rapprochement axial entre le plateau 10 et l'élément 40 se poursuit jusqu'à ce que la totalité, à un jeu fonctionnel près, de la partie annulaire 17 soit logée dans l'orifice 42, comme représenté sur la figure 3. Le chant d'extrémité proximale de la partie tubulaire 43 s'étend alors en affleurement de la surface distale 12B, l'élément 40 étant ainsi assemblé au plus près de la plaque 12.

L'immobilisation axiale de la partie annulaire 17 dans l'orifice 42 est, au moins en partie, liée à la coopération des surfaces tronconiques 17₂ et 43₁, ces surfaces ayant tendance à se coincer l'une contre l'autre à la façon d'un cône Morse. En pratique, cette coopération sur une dimension axiale comprise entre 5 et 10 mm et avec un demi-angle α de l'ordre de 6° est suffisante pour permettre un assemblage satisfaisant en terme de centrage et de résistance mécanique. La dimension axiale de la partie annulaire 17 est donc avantageusement comprise entre 5 et 15 mm, de préférence égale à 10 mm.

Avant ou après que le plateau soit ainsi rapporté à l'élément 40, l'extrémité proximale 33 de la tige 30 est axialement introduite dans l'orifice distal 44, de manière centrée sur l'axe 31, jusqu'à ce que son épaulement 35 vienne en butée axiale contre le fond de l'orifice 44, avec son pion 34 s'étendant successivement dans le passage 46 et dans l'orifice proximal 42, comme représenté à la figure 3. En pratique, la tige est ainsi introduite dans l'orifice 44 alors que l'élément de verrouillage 50 est reçu dans le trou 16 du plateau 10 dont la partie 17 est déjà reçue dans l'orifice proximal 42. Par conséquent, la progression axiale de la tige 30 vers le haut nécessite le vissage de son pion 34 dans le trou taraudé 52 de l'élément 50. Pour faciliter ce vissage, l'élément 50 est immobilisé en rotation autour de son axe 51₁, dans l'orifice 16, par complémentarité de formes entre, d'une part, une renflement 53 s'étendant axialement en saillie vers le bas depuis la surface distale du corps 51, et, d'autre part, une cavité 48 creusée depuis la surface de fond de l'orifice proximal 42, dans une paroi de l'élément 40 formant transition entre les parties proximale 43 et distale 45 de cet élément. Lorsque ce renflement 53 et cette cavité 48 sont en prise l'un dans l'autre, l'élément 50 est immobilisé en rotation autour de l'axe 11 et le trou traversant 52 est aligné de manière coaxiale avec le passage 46, ce qui facilite les manipulations du chirurgien pour introduire et visser la partie de tige proximale 33 dans successivement l'orifice distal 44, le passage 46 et le trou 52.

Ce vissage se poursuit jusqu'à ce que l'élément 50 soit fermement serré sur le pion fileté 34, de sorte que la paroi de l'élément 40 séparant ses parties proximale 43 et distale 45 est enserrée entre l'épaulement 35 de la tige et la surface distale du corps 51, comme représenté à la figure 3. Avantageusement, ce serrage de l'élément 50 contribue également à immobiliser axialement le plateau 10 et l'élément 40. A cet effet, le corps 51 est muni, à son extrémité proximale, d'un rebord périphérique 54 radialement saillant depuis la surface extérieure du corps, tandis qu'une rainure complémentaire 18 est prévue suivant la périphérie de l'orifice 16, au niveau de son débouché proximal. Lorsque l'élément 50 est reçu dans l'orifice 16, le rebord 53 est logé dans la rainure 18 de manière à presser axialement la plaque 12 vers l'élément 40 lors du serrage de l'élément 50.

Ainsi, en une seule opération de vissage de la partie de tige proximale 33, le chirurgien verrouille en position le plateau 10, la tige 30 et l'élément adaptateur 40 dans une configuration d'assemblage permettant leur implantation ultérieure dans le tibia. Le patin 20 est rapporté par la suite.

Divers aménagements et variantes à l'implant tibial décrit ci-dessus sont par ailleurs envisageables. A titre d'exemples :
- la géométrie du patin 20 n'est pas limitée à celle représentée ; de même, le patin mobile 20 peut être remplacé par un patin fixe, c'est-à-dire à même d'être porté de manière immobile sur la surface proximale plateau 10;
- le nombre de crans 47 répartis suivant la périphérie de la partie proximale 43 de l'élément 40 peut être supérieur ou inférieur à celui considéré ci-dessus ; de même, ces crans peuvent, en variante, être réalisés sur la surface intérieure de la partie 43, autrement dit dans l'orifice proximal 42, moyennant des aménagements correspondants des ailettes 15 ; et/ou
- la partie distale 32 de la tige 30 ne s'étend pas nécessairement dans le prolongement rectiligne de la partie proximale 33 ; cette partie distale peut être incurvée sur la longueur, l'axe tibial 31, par rapport auquel le décalage radial avec l'axe tibial 11 est réalisé, correspondant alors à l'axe central de la partie proximale de la tige.

## Revendications

1. Implant tibial (1), comprenant :
- un plateau (10) qui définit un premier axe tibial (11) autour duquel le plateau s'étend transversalement et qui comporte une plaque (12) délimitant une surface distale (12B) qui est adaptée pour être appuyée fixement contre l'extrémité supérieure du tibia d'un patient et depuis laquelle une partie (17) du plateau s'étend directement en saillie suivant sensiblement le premier axe tibial,
- une tige (30) qui définit un second axe tibial (31) autour et le long duquel au moins une partie proximale (33) de la tige s'étend et qui comporte une partie distale (32) adaptée pour être introduite dans le canal médullaire du tibia, et
- un élément adaptateur (40) qui est adapté pour être interposé fixement entre le plateau et la tige et qui comporte des parties proximale (43) et distale (45) à même d'être respectivement assemblées au plateau et à la tige, de manière respectivement centrée sur les premier et second axes tibiaux, avec ces deux axes sensiblement parallèles et éventuellement décalés l'un par rapport à l'autre,
la partie proximale (43) de l'élément adaptateur (40) délimitant un orifice proximal (42) de réception de ladite partie (17) du plateau (10), partie qui est sensiblement complémentaire de cet orifice proximal et qui est adaptée pour être logée sensiblement en totalité dans cet orifice proximal lorsque le plateau et l'élément adaptateur sont assemblés l'un à l'autre,
**caractérisé en ce que** la partie proximale (43) de l'élément adaptateur (40) est munie de moyens d'indexage angulaire (47), autour du premier axe tibial (11), par rapport au plateau (10), adaptés pour régler de manière discrète le positionnement à la fois médio-latéral et antéro-postérieur du second axe tibial (31) par rapport au premier axe tibial (11) lorsque ladite partie (17) du plateau est logée dans l'orifice proximal (42) de l'élément adaptateur.

2. Implant suivant la revendication 1, **caractérisé en ce que** la position angulaire, autour du premier axe tibial (11), de l'élément adaptateur (40) par rapport au plateau (10) est indexable par les moyens d'indexage (47) avec un pas angulaire strictement inférieur à 180°, tel qu'un pas de 15°.

3. Implant suivant l'une des revendications 1 ou 2, **caractérisé en ce que** les moyens d'indexage angulaire comportent des crans (47) régulièrement répartis suivant la périphérie, extérieure ou intérieure, d'une paroi tubulaire (43) de l'élément adaptateur (40), délimitant intérieurement l'orifice proximal (42).

4. Implant suivant l'une quelconque des revendications précédentes, **caractérisé en ce que** les moyens d'indexage angulaire (47) sont adaptés pour coopérer par complémentarité de formes avec au moins un repère (15) du plateau (10), qui s'étend directement en saillie depuis la surface distale (12B) de la plaque (12), de manière à immobiliser en rotation, autour du premier axe tibial (11), l'élément adaptateur (40) par rapport au plateau lorsque ladite partie (17) du plateau est logée dans l'orifice proximal (42).

5. Implant suivant l'une quelconque des revendications précédentes, **caractérisé en ce que** ladite partie (17) du plateau (10) présente une forme essentiellement annulaire, centrée sur le premier axe tibial (11).

6. Implant suivant l'une quelconque des revendications précédentes, **caractérisé en ce que** la dimension axiale de ladite partie (17) du plateau (10) est inférieure à 15 mm, de préférence égale à 10 mm.

7. Implant suivant l'une quelconque des revendications précédentes, **caractérisé en ce que** la partie distale (45) de l'élément adaptateur (40) délimite un orifice distal (44) de réception de la partie proximale (33) de la tige (30), lequel orifice distal débouche dans l'orifice proximal (42) en ménageant un passage (46) dans lequel l'extrémité proximale (34) de la tige est à même de s'étendre jusqu'à l'orifice proximal (42) lorsque la tige et l'élément adaptateur sont assemblés l'un à l'autre.

8. Implant suivant la revendication 7, **caractérisé en ce qu'**il comprend en outre un élément (50) de verrouillage du plateau (10), de la tige (30) et de l'élément adaptateur (40) assemblés les uns aux autres, lequel élément de verrouillage est adapté pour être au moins en partie introduit dans l'orifice proximal (42) pour s'y accoupler à l'extrémité distale (34) de la tige.

9. Implant suivant la revendication 8, **caractérisé en ce que** la plaque (12) du plateau (10) délimite un orifice traversant (16) qui s'étend suivant le premier axe tibial (11) et dans lequel l'élément de verrouillage (50) est adapté pour être introduit pour s'étendre jusqu'à l'orifice proximal (42) lorsque le plateau et l'élément adaptateur (40) sont assemblés l'un à l'autre.

10. Implant suivant l'une des revendications 8 ou 9, **caractérisé en ce que** l'élément de verrouillage (50) est muni de moyens (53) d'immobilisation en rotation autour du premier axe tibial (11) par rapport au plateau (10), adaptés pour coopérer avec des moyens associés (48) prévus sur l'élément adaptateur (40) lorsque le plateau et l'élément adaptateur sont assemblés l'un à l'autre.
